# EUROPEAN PATENT SPECIFICATION

(11) **EP 4 313 202 B1**
(45) Date of publication and mention of the grant of the patent: **30.07.2025**
(21) Application number: 22776730.8
(22) Date of filing: 25.03.2022
(51) Int. Cl.: A61M 1/16, A61J 3/00, C02F 1/00, A61M 1/28

(54) **MEDICAMENT PREPARATION DEVICES, METHODS, AND SYSTEMS**
VORRICHTUNGEN, VERFAHREN UND SYSTEME ZUR HERSTELLUNG VON ARZNEIMITTELN
DISPOSITIFS, PROCÉDÉS ET SYSTÈMES DE PRÉPARATION DE MÉDICAMENT

(30) Priority: 26.03.2021 US 202163166396 P
(43) Date of publication of application: 07.02.2024
(73) Proprietor: NxStage Medical, Inc., Lawrence, MA 01843 (US)
(72) Inventor: FRIEDERICHS, Goetz, Waltham, MA 02451 (US); YANTZ, Gregory, Waltham, MA 02451 (US)
(74) Representative: Freischem & Partner Patentanwälte mbB
(86) International application number: PCT/US2022/021955
(87) International publication number: WO 2022/204520

(56) References cited:
- WO-A1-2020/237033
- US-A1- 2017 319 768
- US-A1- 2019 151 526
- US-A1- 2019 262 522
- US-A1- 2019 262 526
- US-A1- 2020 009 308
- US-A1- 2020 171 230

## Description

### Cross-Reference to Related Applications

This application claims the benefit of U.S. Provisional Application No. 63/166,396 filed March 26, 2021.

### Background

The disclosed subject matter relates generally to devices, methods, systems, improvements, and components for preparing medicaments and making medicament available for use by a consumer, for example, a dialysis cycler.

Peritoneal dialysis is a mature technology that has been in use for many years. It is one of two common forms of dialysis, the other being hemodialysis, which uses an artificial membrane to directly cleanse the blood of a renal patient. Peritoneal dialysis employs the natural membrane of the peritoneum to permit the removal of excess water and toxins from the blood.

In peritoneal dialysis, sterile peritoneal dialysis fluid is infused into a patient's peritoneal cavity using a catheter that has been inserted through the abdominal wall. The fluid remains in the peritoneal cavity for a dwell period. Osmotic exchange with the patient's blood occurs across the peritoneal membrane, removing urea and other toxins and excess water from the blood. Ions that need to be regulated are also exchanged across the membrane. The removal of excess water results in a higher volume of fluid being removed from the patient than is infused. The net excess is called ultrafiltrate, and the process of removal is called ultrafiltration. After the dwell time, the dialysis fluid is removed from the body cavity through the catheter.
It is referred to US 2019/0262522 A1 and WO 2020/237033 A1 as prior art.

### Summary

A method of generating a custom mini batch of dialysate according to the present invention comprises the technical features as defined in independent claim 1.

Methods, device, and systems for preparing medicaments such as, but not limited to, dialysis fluid are disclosed. In embodiments, medicament is prepared at a point of care (POC) automatically using a daily sterile disposable fluid circuit, one or more concentrates to make batches of medicament at the POC. The dialysis fluid may be used at the POC for any type of renal replacement therapy, including at least peritoneal dialysis, hemodialysis, hemofiltration, and hemodiafiltration.

In embodiments, peritoneal dialysis fluid is prepared at a point of use automatically using a daily sterile disposable fluid circuit and one or more long-term concentrate containers that are changed only after multiple days (e.g. weekly). The daily disposable may have concentrate containers that are initially empty and are filled from the long-term concentrate containers once per day at the beginning of a treatment.

Embodiments of medicament preparation, devices, systems, and methods are described herein. The features, in some cases, relate to automated dialysis such as peritoneal dialysis, hemodialysis and others, and in particular to systems, methods, and devices that prepare peritoneal dialysis fluid in a safe and automated way at a point of care. The disclosed features may be applied to any kind of medicament system and are not limited to dialysis fluid.

In embodiments, a system that prepares a medical fluid is configured in such a manner that it outputs the medical fluid to a consuming process (for example, a peritoneal dialysis cycler) wherein the consuming process does not distinguish between the system that prepares the medical fluid and pre-packaged bags of dialysate. This allows embodiments of the presently disclosed system for preparing the medical fluid to be used with any type of a cycler, without any special customization or modification of the cycler.

Objects and advantages of embodiments of the disclosed subject matter will become apparent from the following description when considered in conjunction with the accompanying drawings.

### Brief Description of the Drawings

Embodiments will hereinafter be described in detail below with reference to the accompanying drawings, wherein like reference numerals represent like elements. The accompanying drawings have not necessarily been drawn to scale. Where applicable, some features may not be illustrated to assist in the description of underlying features.
Fig. 1A shows a system for preparing a ready-to-use medicament from concentrated medicament and water according to embodiments of the disclosed subject matter.
Fig. 2A shows a flow chart of a method for checking the concentration and/or conductivity of medicament according to embodiments of the disclosed subject matter.
Fig. 2B show a flow chart of a method for preparing a ready-to-use medicament according to embodiments of the disclosed subject matter.
Fig. 3 shows a system for generating purified water for the system and method of Fig. 1A according to embodiments of the disclosed subject matter.
Fig. 4A shows configurations of the systems providing water to a mixing container according to embodiments of the disclosed subject matter.
Figs. 5A and 5B show configurations of systems providing various types of medicament concentrate to the mixing container according to embodiments of the disclosed subject matter.
Fig. 6A shows configurations of the systems mixing the content of the mixing container according to embodiments of the disclosed subject matter.
Fig. 7 shows configurations of the system draining the content of the mixing container according to embodiments of the disclosed subject matter.
Figs. 8A and 8B show configurations of the systems providing the content of the mixing container to a consumer of the content according to embodiments of the disclosed subject matter.
Fig. 9 shows a computer system that may describe the functions and elements of a controller as described herein and in accordance with the embodiments of the disclosed subject matter.

### Detailed Description

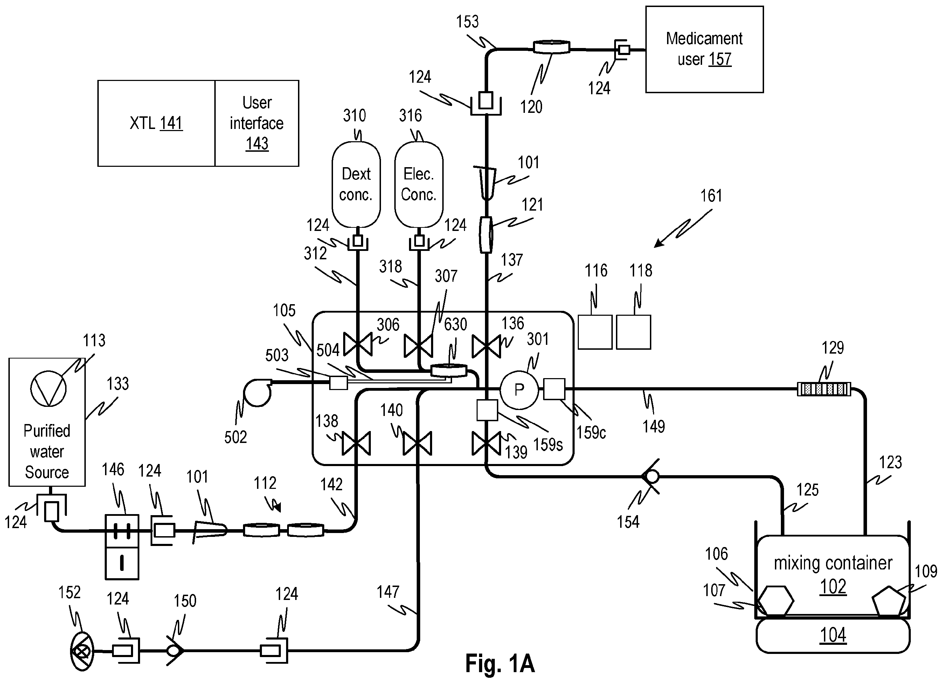

Fig. 1A shows an embodiment of a system that uses water and up to two concentrated medicaments (also referred to as "medicament concentrates" or "concentrates") in containers 310 and 316 to make a therapeutic fluid that can be used for treatment according to embodiments of the disclosed subject matter. In embodiments, the concentrated medicament in container 310 is an osmotic agent. In embodiments, the osmotic agent includes concentrated dextrose solution. In other embodiments, the osmotic agent includes concentrated glucose solution. In embodiments, the concentrated medicament in container 316 is an electrolyte concentrate.

Each of the containers 310 and 316 may be connected to fluid lines 312 and 318 via a connector 124, as shown. However, it is also possible that each or one of the containers are pre-connected to the fluid lines 312 and 318, thus avoiding connectors 124. Osmotic concentrate container 310 is fluidly connected to osmotic fluid line 312. An optional one-way check valve (not illustrated) may be provided on fluid line 312. Similarly, electrolyte concentrate container 316 is fluidly connected to electrolyte fluid line 318 and may include one-way check valve (not shown). These one-way check valves are optional and may be omitted. One-way check valves may be beneficial when multiple batches of medicament are made without changing concentrate containers, as they prevent contamination from reaching the concentrated medicament containers 310 and 316. Osmotic fluid line 312 is controlled by osmotic valve 306. Electrolyte fluid line 318 is controlled by electrolyte valve 307. These valves can open and close under the control of a controller, such as controller 141.

The concentrate lines 312 and 318 pass through the valves 306 and 307, respectively. The lines and then continue and merge before they passed through a single sterilizing filter 630. The sterilizing filter 630 may be a 0.2 µm filter, or similar. In an embodiment, the filter has a pore size of 0.2 micrometers. As can be appreciated from Fig. 1A, sterilizing filter 630 is fluidly connected to pressure sensor 301 and the peristaltic pump 129. It will be appreciated that whenever of the peristaltic pump 129 operates (rotates to thereby convey fluid through a pumping tube segment) it generates pulsatile pressure in fluid line 149. This pulsatile pressure acts on the membrane of sterilizing filter 630. Because there are no valves disposed between peristaltic pump 129 and the sterilizing filter 630, filter 630 always experiences this pulsatile pressure when the peristaltic pump 129 operates. Providing a single sterilizing filter 630 which sterilizes both the content of the osmotic concentrate container 310 as well as the contents of the electrolyte concentrate container 316 reduces the cost of the disposable unit 161 but exposes filter 630 to the pulsatile pressure of the pump 129.

To mitigate the risk of damaging the membrane of sterilizing filter 630 in such a way that pathogens could pass through the membrane, a filter integrity test configuration is provided. An air pressure pump 502 is provided and connected through a connector 503 to a pressure test line 504 which further connects to filter 630. In embodiments, connector 503 may include a pressure transducer and may provide an output to controller 141.

Testing of sterilizing filter 630 using pressurized air testing can be done in various ways, for example, a bubble point test can be performed. Bubble point refers to the pressure at which the first flow of air through a liquid saturated membrane of filter 630 first occurs and it is a measure of the largest pore-throat in the membrane. The bubble point method is based on the principle that the critical pressure of an airflow applied across the thickness of a membrane evacuates the fluid trapped in the pore with the largest pore-throat. Therefore the applied pressure must exceed the capillary pressure of the fluid in the largest pore-throat. In testing, the membrane is saturated with a liquid. The gas pressure on the upstream face of the saturated membrane is then increased to a pressure when the first air bubble passes through the largest pore-throat in the saturated fabric. An expected bubble pressure of an intact filter membrane may be stored, and a new measurement of bubble pressure may be taken periodically (once the membrane of filter 630 is wetted with a fluid, whether a concentrated medicament, water, or a mixture of water and concentrated medicament). The new measurement may be compared with the stored expected bubble pressure, and if the new measurement is below the expected value by a threshold amount, a filter failure is signaled.

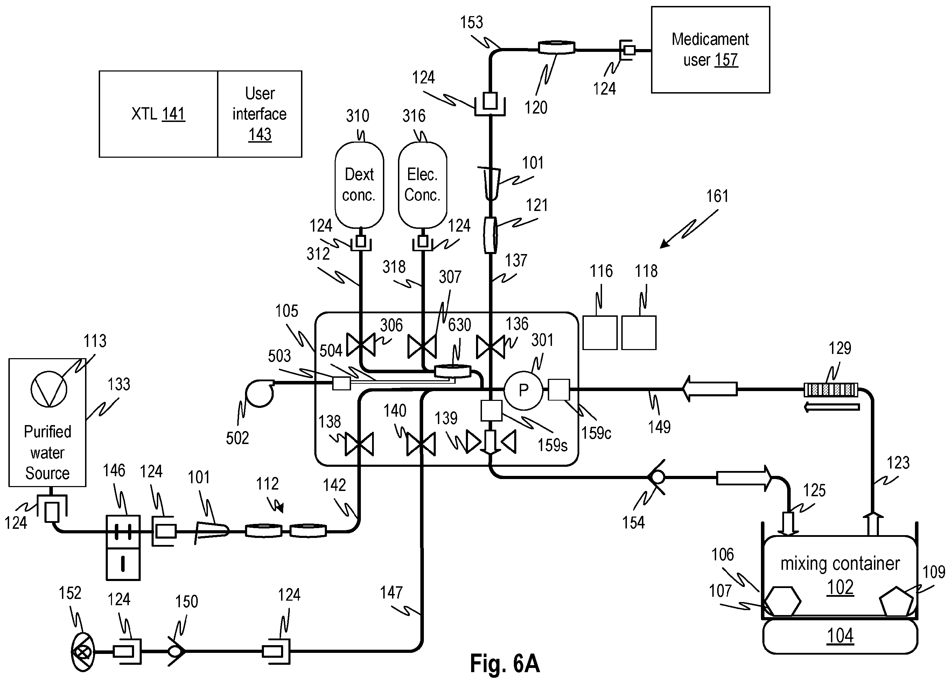

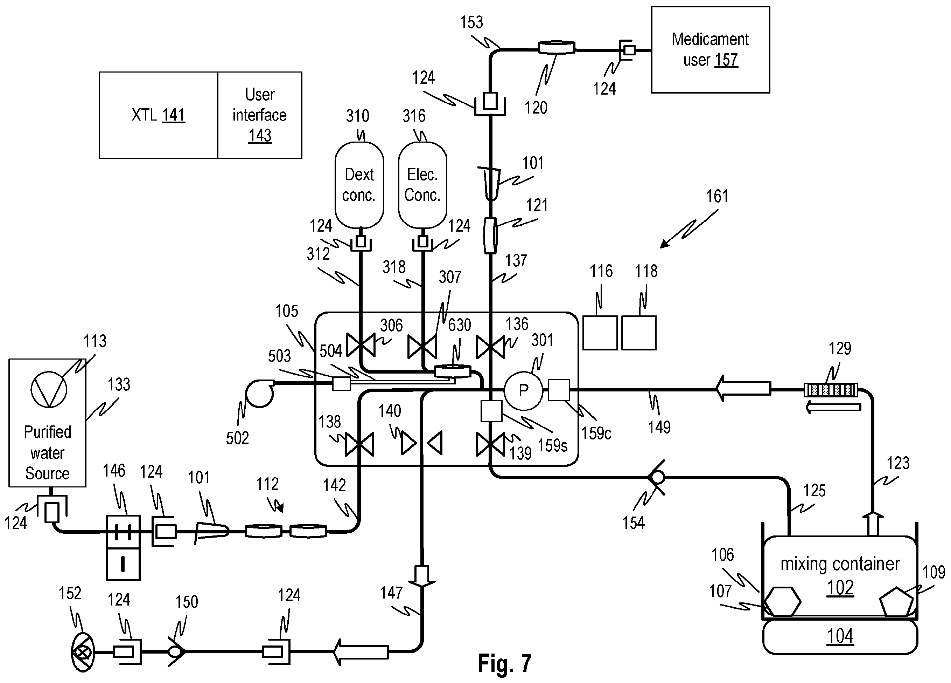

If a filter failure is signaled, the contents of the mixing container 102 may be deemed to be unusable and may be drained and discarded by placing the fluid circuit into the configuration illustrated in Fig. 7. In an embodiment, the filter integrity test takes place after each of the osmotic concentrate and the electrolyte concentrate are provided into the mixing container 102, and after water is mixed with the concentrate in the mixing container 102. This way, the filter failure can be detected and signaled earlier in the process so that remedial steps can be taken. In embodiments, a filter integrity test takes place during the time that the contents for the mixing container 102 are being mixed which is illustrated in the configuration of Fig. 6A. In embodiments, the filter integrity test takes place after the final amount of water has been conveyed into the mixing container 102, but before the mixing process is complete.

In embodiments, a pressure decay test can be done instead of a bubble test to test the integrity of the membrane of filter 630, where fluid is pumped across the membrane and the pressure drop measured and compared with a pressure drop representative of an intact filter or pressure is increased on one side, pumping stopped, and the rate of decay of pressure compared to a predefined curve representative of an intact filter. In other embodiments, the filter 630 is housed in an air-tight container and the container is pressurized to a level that is below the expected bubble point, but high enough to guarantee that the membrane is sterilizing grade. The filter 630 may have air vents so this pressurizes the membrane. The rate of (air) pressure decay is then measured and if the decay rate is greater than a predefined threshold rate, the filter is indicated to have failed.

Other means of testing filter integrity may be used, for example, concentrates 310 and/or 316 can include a large-molecule excipient whose presence can be detected using automatic chip-based analyte detection (e.g., attachment of fluid samples to selective fluorophore after flowing through the filter and optical detection after concentration).

Still referring to Fig. 1A, a purified water source 133 with a water pump 113 supplies highly purified water through a connector 124 through a water line 142. The water line 142 has a non-reopenable clamp 146, another connector 124, a manual tube clamp 101, and a pair of redundant 0.2-micron sterilizing filters 112, as shown. In embodiments, different types of sterilizing filters may be used, and not limited to 0.2 micron, or to two redundant filters. For example, a single filter may be used, and a testing protocol provided to ensure that the filter does not fail before replacement.

A water inlet clamp 138, batch release clamp 136, and a conductivity sensor clamp 140 are controlled by a controller 141, which may be operatively coupled to a user interface 143, which may include a visual and/or audible output and various devices for receiving user input. The controller 141 controls the pinch clamps and a peristaltic pump 129 to make a batch of diluted concentrate in a mixing container 102 by diluting medicament concentrate (e.g., dialysis fluid concentrate) in the mixing container 102. The mixing container 102 is supplied empty and permanently connected to a fluid circuit that includes fluid lines 149, 123, and 125. In embodiments, the mixing container 102 may be detached initially, and attached to the rest of the fluid circuit which forms the disposable component 161 prior to the preparation of medicament.

A pressure sensor 301 is provided in the flow path as shown and outputs a signal representative of the pressure in the fluid lines that are fluidly connected to the pressure sensor. This pressure signal may be provided to controller 141.

In embodiments, the pressure sensor 301 may be used, at least partially, to perform the filter integrity tests described above. The pressure sensor 301 is in fluid communication with the filter 630 and can be used to measure the pressure on one side of the membrane of the filter 630, to perform e.g., a pressure decay test.

The mixing container 102 may be a part of a disposable unit or component 161 that is replaced regularly, such as with each batch, every day, every week, or every month. In an embodiment, the mixing container 102 is empty initially when the disposable component 161 is connected to the system.

The mixing container 102 may be made of a flexible material, such as a polymer so its shape is not rigid. To provide support for the mixing container 102, it is held by a tub 106 which is sufficiently rigid to support the mixing container 102 when it is full of fluid. A leak sensor 107 is provided in the tub 106 and it detects leaks into the tub 106 while a temperature sensor 109 may also be provided in or on the tub 106 and it detects the temperature of the fluid in the mixing container 102. A warmer 104 may be provided as shown to provide heat to tub 106, but the warmer 104 may be omitted if another heater exists elsewhere in the system. Note that the concentrates 310 and 316 that will be supplied to the mixing container 102 may be used for making any type of medicament, not just dialysis fluid.

A cracking pressure check valve 154 is provided on inlet line 125. The check valve 154 prevents flow in line 125 out of mixing container 102 and allows flow into mixing container 102 only when the cracking pressure is overcome. The cracking pressure may be selected at 3.5 PSI (1 PSI = 6894.76 Pa) in embodiments. As described in greater detail below, using the check valve 154 allows for different fluid line configurations. In addition to the check valve 154, a controllable valve (supply line clamp 139) is provided on the supply line 125.

Likewise, a check valve may be added to the concentrate supply lines 312 and 318 (not shown), preventing back flow of concentrate into the containers 310 and 316. In embodiments, this allows for the safe preparation of multiple batches of diluted medicament from the same containers of concentrate, as back flow (which is undesirable) into the concentrate container is prevented.

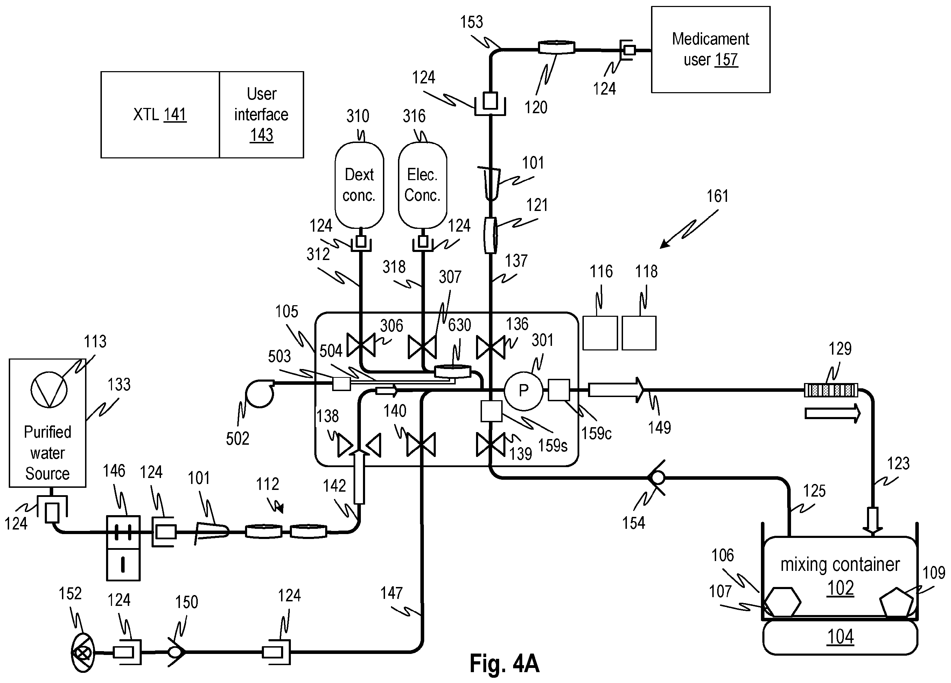

To supply water to mixing container 102, pump 129 runs to move the water from water line 142 to supply line 123 and mixing container 102 while valve 138 is open, as shown in Fig. 4A. The water may be provided by the purified water source 133 and its pump 113 at a pressure that is below the cracking pressure of check valve 154, so that no water will flow through inlet 125, but only through inlet 123, even if valve 139 were opened. But during normal water filling operation, valve 139 may remain closed, as shown in Fig. 4A.

The mixing container at 102 may be part of the disposable unit 161. Included in a disposable unit 161 are the two concentrate supply lines 312 and 318, transfer line 149, water source line 142, drain conductivity line 147, medicament supply line 153 and the mixing container 102 with its respective fill lines 123 and 125. The disposable unit 161 is permanently interconnected up to and including an end of each of the connectors 124, through which various other components can be connected (including the medicament user 157, the purified water source 133, the osmotic agent concentrate 310, the electrolyte concentrate 316, and the drain connection 152). Also included in the disposable unit 161 may be the check valve 154 that has a predefined cracking pressure (e.g., 3.5 PSI). The disposable unit 161 may also include the sterilizing filter 630 which further includes a test line 504 with a connector 503. Connector 503 will be connected to an airline output of air pressure pump 502. The disposable unit 161 can be connected to check valve 150 which prevents back flow in the drain line 147.

A door lock 116 is provided adjacent a user interface door 105 to lock the user interface door. A physical door 105 that opens encloses and provides access to the interior of the fluid preparation system may have a user interface on it which may be a part of user interface 143. A door sensor 118 detects whether the door lock is in an open or a locked position to ensure that all clamps and the peristaltic pump actuators are fully engaged with the disposable fluid circuit.

The door sensor 118 may include a plunger which is pressed in when the door is closed and outputs an electrical signal to indicate whether or not the door is closed. In other embodiments, the door sensor 118 may include a magnetic reed switch which detects the presence or the absence of a magnet which is located on the door 105 at a location which is detectable by the reed switch. Purified water flows into the disposable circuit where a pair of 0.2-micron filters (also in the disposable unit 161) are located to ensure that any touch contamination is prevented from flowing into the disposable circuit. An optional sterilizing filter 120 may be provided in a user medicament supply line 153. The sterilizing filter 120 may be a 0.2 micron filter. The mixing container 102 of the disposable unit 161 may have sufficient volume for a single treatment or in embodiments, multiple treatments. To make a batch of dilute concentrate, water is pumped into the mixing container 102 which contains concentrate sealed in it as delivered.

A conductivity/temperature sensor 159c (control) is provided on or fluidly connected to transfer line 149, as shown. The sensor 159c forms a part of the disposable component 161 and may be a type of a conductivity and temperature sensor that allows fluid to flow through it while it detects the temperature and/or the conductivity of the fluid flowing therethrough. In embodiments, the conductivity reading is calibrated by the measured temperature. The output of the sensor 159c is provided to a controller, such as controller 141.

A second conductivity/temperature sensor 159s (system) is provided on or fluidly connected to inlet line 125, as shown, and also forms a part of the disposable unit 161. The physical structure of sensor 159s may be the same or similar to that of sensor 159c. The output signal from sensor 159s may be provided to controller 141.

Both of sensors 159c and 159s are shown as being behind the door 105 of the system. In this configuration, the internal environment of the system provides temperature stability that isolates the sensors from the outside environment and may improve measurement accuracy. In embodiments, the sensors 159c and 159s physically mate to internal structures behind the door 105 and thereby allow the system to detect whether the sensors 159c and/or 159s are present. In embodiments, there are multiple different configurations of disposable element 161, and some configurations do not include one or both of the sensors, and the position of the sensors on the disposable element 161, as shown, allows the system to detect and in certain situations to self-configure to adapt based on the presence or absence of the two sensors. In embodiments, one or more separate optional conductivity sensors can be provided on line 147 (not shown in the figure) that can measure conductivity and/or temperature of fluid flowing through drain line 147 toward drain connection 152. If sensors 159c and 159s are not detected on the disposable component 161, the system may adapt and rely on the (unillustrated) conductivity/temperature sensor(s) on the drain line 147 to measure conductivity of fluid.

The medicament output line 137 may include an optional air removal filter 121. The air removal filter 121 may be a 1.2 µm filter which removes air from the medicament supplied through medicament supply line 137 before it reaches the medicament user 157.

A check valve 150 in drain conductivity line 147 ensures the flow does not reverse to safeguard against contamination in the medicament or water lines or other sterile fluid circuits.

Because conductivity/temperature sensor(s) 159c and 159s are provided on the fluid circuit as shown in Fig. 1A, it is possible to control various valves to establish a loop through which fluid from the mixing container flows while the conductivity of the fluid is tested, but little, if any, fluid is wasted. Various examples of a physical configuration of the fluid circuit are shown in Fig. 6A. Valves 136, 138, 140, 306, 307 are closed while valve 139 is opened and peristaltic pump 129 operates to draw fluid through inlet line 123 and to circulate the fluid along line 149 through or past sensor 159c and then through or past sensor 159s. The fluid is pumped at a pressure above the cracking pressure of valve 154 so the fluid continues to flow back into the mixing container 102 through inlet line 125. This process continues as long as the valves remain closed and the peristaltic pump 129 operates. It will be understood that during this process the contents of the mixing container 102 is mixed, which helps provide stable reading of conductivity in the sensors 159c and 159s.

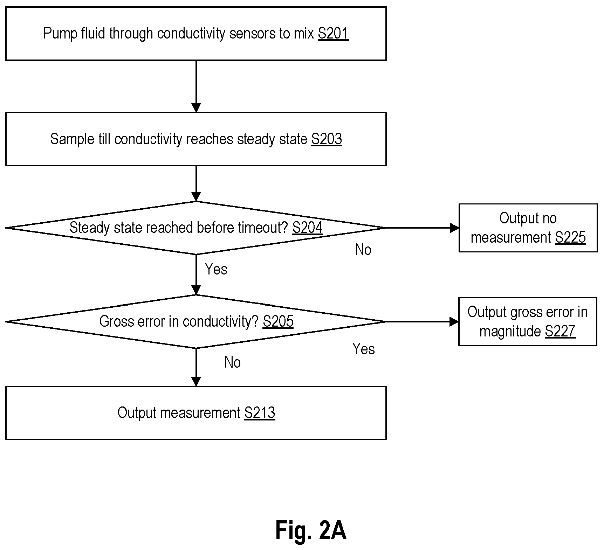

Referring now to Fig. 2A, at S201 the fluid circuit will be configured as shown in Fig. 6A. In this procedure multiple consecutive measurements are made of conductivity and temperature at consecutive times so that the conductivity is measured and a trend can be observed to determine whether the conductivity has reached a steady state value. At each measurement time, a separate value can be produced from sensor 159c and a separate value can be produced from sensor 159s. By comparing the two values, it is possible to confirm whether the sensors are within some expected range. If the two sensor readings are within an expected range of values, it may be determined that both sensors are working correctly. On the other hand, if the values differ by some predetermined threshold, the process may conclude that one or both of the sensors are malfunctioning at S205 and output a gross error warning at S227.

In embodiments, only a single sensor 159c or 159s may be provided. Multiple readings may be taken from such a single sensor, and compared to an expected value. If one or more of the multiple readings differ from the expected value by a predetermined threshold, another gross error in conductivity may be detected at output as a warning or an error message in S227.

The conductivity is sampled until it reaches a steady state at S203. If the steady state is reached before of time out, another comparison to an expected value can be performed at S205 to determine whether a gross error in conductivity has occurred. If the steady state is not reached before the time out, no measurement is output at S225.

If the steady state is reached and the value that is read does not indicate a gross error, a measurement is output and may be provided to controller 141. The measurement may be an average between the values output from sensor 159c and sensor 159s. In embodiments, the measurement that is output may be a value that is output from only one of those sensors, or it may be a time average of multiple readings from that one sensor.

Note that temperature-compensated conductivity is intended to refer to a number that is proportional to concentration and may be determined in various ways including but not limited to a lookup table and a formula. For the remainder of this disclosure a reference conductivity the reference may be understood to mean temperature-compensated conductivity or an actual calculation of concentration. That is, the temperature-compensated conductivity may be a value that is generated by the controller by multiplying the measured conductivity with a value that represents the rate of change of concentration with temperature. In other embodiments, the controller 141 may calculate a concentration directly using a look-up table or formula.

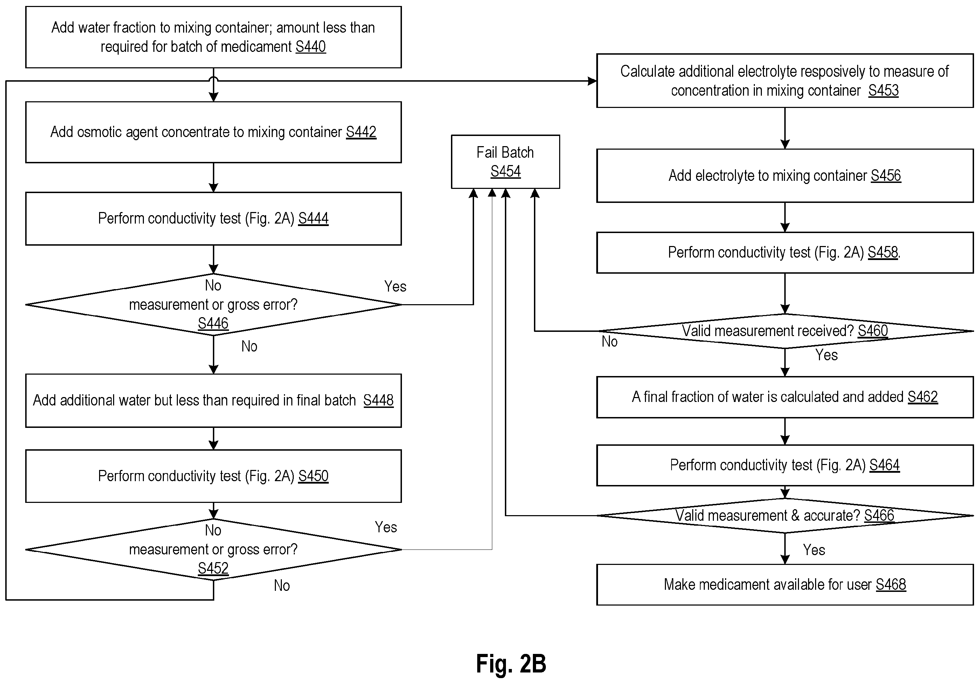

Fig. 2B shows a flow chart for a procedure that may be executed by the controller 141 with respect to the embodiment of Fig. 1A to generate medicament. The procedure of Fig. 2B incorporates the procedure of Fig. 2A by the reference to "conductivity test" described with reference to the procedure of Fig. 2A. When the conductivity test is referenced it means the procedure of Fig. 2A is entered and upon exiting proceeds to the next procedure element in Fig. 2B.

At S440, water is added to the mixing container 102 in an amount that is a fraction of what is determined (or expected) to be required for a complete batch of medicament. The amount of fluid conveyed at S10 may be a fraction of the total estimate required for a sufficient level of dilution, such as 50% of the expected total water volume.

Water is added by pumping it into the mixing container 102 from the purified water source 133. This is done by placing the system in the configuration of Fig. 4A. The water pump 113 and the peristaltic pump 129 are activated for a predefined number of cycles or a predefined time interval, resulting in a quantify of water being conveyed along water line 142, through opened valve 138, through transfer line 149, through peristaltic pump 129 and through connector line 123 into mixing container 102.

At this time, a filter integrity test of filter 630 may be performed, as the water filling process will have wetted at least one side of the membrane of filter 630. If the filter integrity test returns a filter error, the process may end at S454 with a failed batch. In embodiments the filter integrity test may be omitted in S440 and performed in a later step.

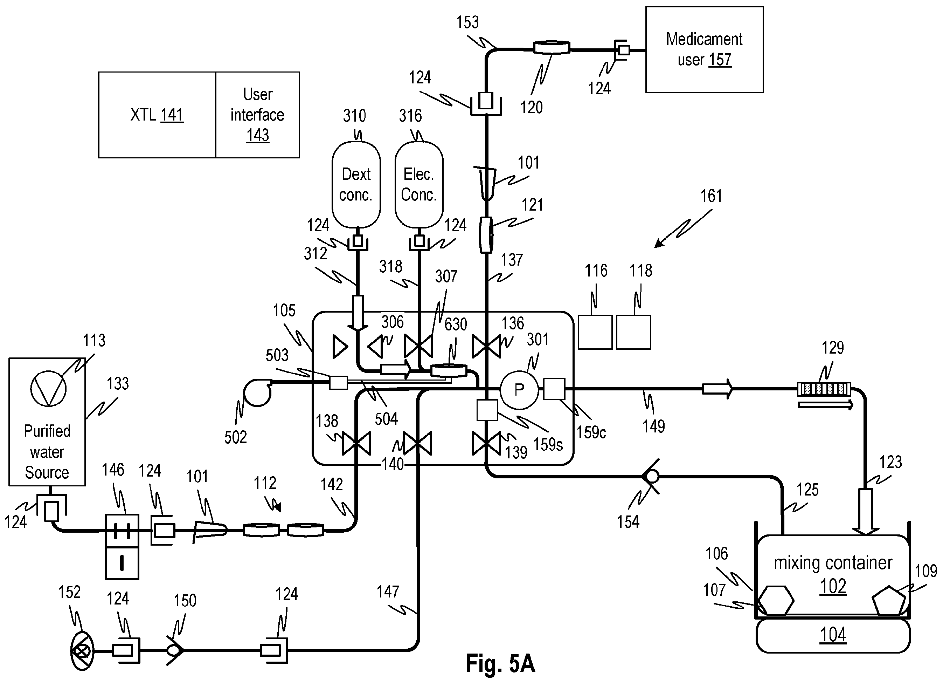

In an embodiment, the entire quantity of osmotic agent concentrate is transferred from container 310 to the mixing container 102 at S442. The fluid circuit takes on the configuration shown in Fig. 5A by controlling valve 306 to open and operating the peristaltic pump 129 in the forward direction as shown by the arrow under the pump 129 in Fig. 5A. Then the contents of the mixing container 102 are mixed by placing the fluid circuit into the configuration shown in Fig. 6A. In other embodiments, less than the entire quantity of osmotic agent concentrate from container 310 is conveyed to the mixing container 102, leaving a quantity of the concentrate in the container 310 sufficient for making additional batches of dialysate in the future.

At this time, a filter integrity test of filter 630 may be performed, as the water filling process will have wetted at least one side of the membrane of filter 630 and the osmotic agent passing through filter 630 will have completely wetted the filter membrane. If the filter integrity test returns a filter error, the process may end at S454 with a failed batch. In embodiments the filter integrity test may be omitted in S442 and performed in a later step.

The conductivity test described above and illustrated in Fig. 2A is then performed at S444. If an output of gross error or no measurement is received at S446, then the batch is failed at S454. If a measurement is output control proceeds to S448 and additional water is added to the mixing container 102 short of the final amount required to achieve a batch that is usable for the medicament, and the contents of the mixing container 102 are mixed again as described above.

The conductivity test is performed again at S450 and if an output of gross error or no measurement is received S452 then the batch is failed at S454.

Otherwise, an amount of electrolyte is calculated, based on the conductivity measurement received, is at S453. Because the osmotic agent and the electrolyte concentrate are provided in separate containers 310 and 316, it is possible to generate customized batches of medicament (e.g., dialysate) based on a prescription that is customized for a specific patient. It is also possible to generate smaller quantities of diluted medicament than in a situation where all of the concentrated medicament were to be used at once, which allows for a fast walkup time (e.g., less than 1 hour) so a patient can initiate preparation of medicament and then begin therapy in less than an hour.

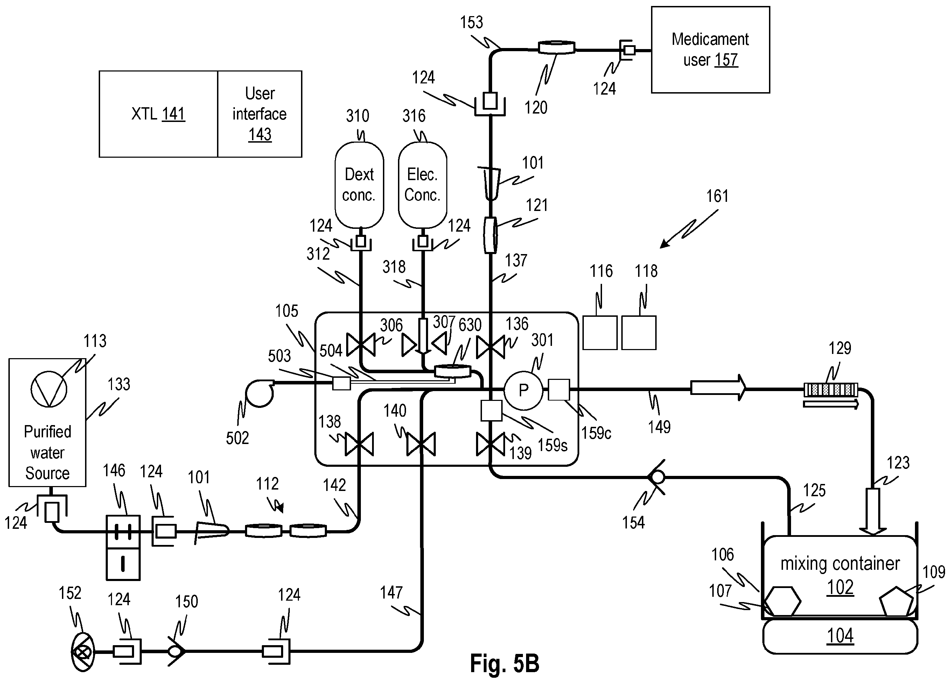

After the calculation, the appropriate amount of electrolyte concentrate is added to the mixing container 102. The fluid circuit is placed into the configuration illustrated in Fig. 5B. As shown in the figure, valve 307 is opened and the peristaltic pump 129 operates in the forward direction to convey the electrolyte concentrate into mixing container 102. It will be appreciated that because the same pump 129 is used for metering both the osmotic agent concentrate from container 310 and the electrolyte concentrate from container 316, the accuracy of the metering is increased, allowing for high precision in establishing the desired custom concentration of the medicament. Once all of the electrolyte concentrate is added, the contents of the mixing container 102 are mixed again as described above.

At this time, a filter integrity test of filter 630 may be performed. If the filter integrity test returns a filter error, the process may end at S454 with a failed batch. In embodiments the filter integrity test may be omitted in S456 and performed in a later step.

At S458 the conductivity test is performed again and if a valid measurement is not received at S460, then the batch is failed at S454. If the measurement is received then at S462 a final fraction of water is then calculated based on the valid measurement and added to the mixing container 102 by placing the fluid circuit into a configuration as shown in Figs. 4C or 4B. Then, the contents of the mixing container 102 are mixed again as described above.

The conductivity test is performed again at S464. If the measurement is valid at S466, then the batch is made available for use at S468. Otherwise, the batch is failed at S454. Before the batch is made available for use, a filter integrity test of filter 630 may be performed. If the filter integrity test returns a filter error, the process may end at S454 with a failed batch.

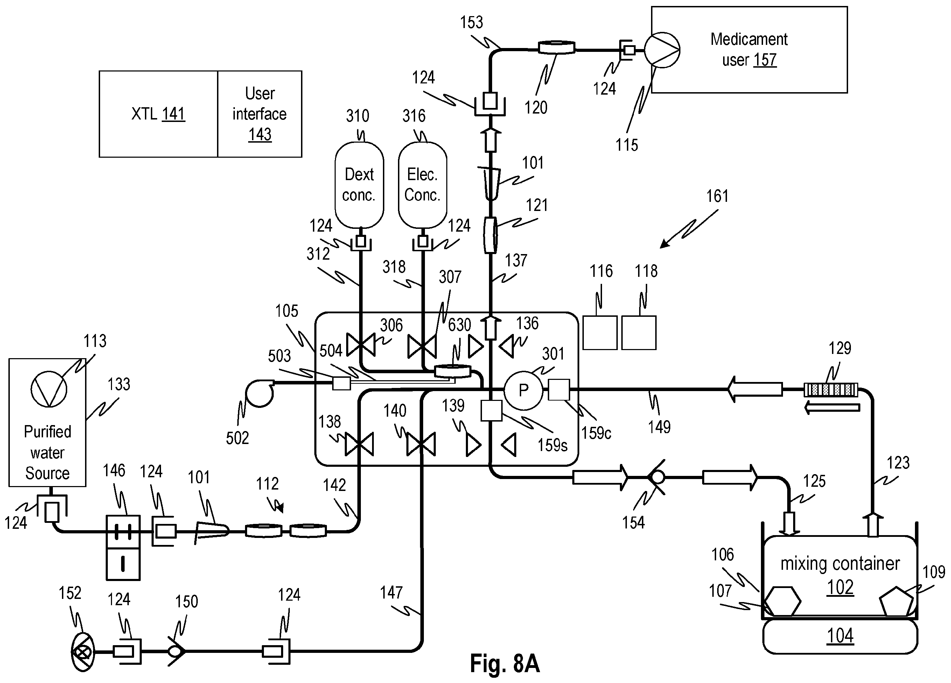

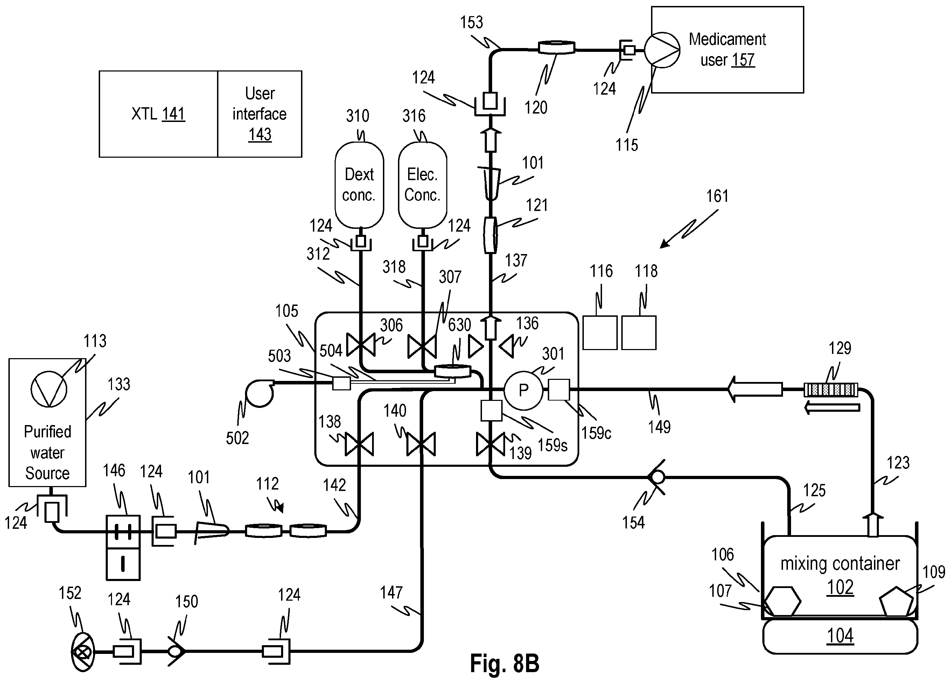

When the batch is made available, the fluid circuit is configured into the configuration shown in Figs. 8A or 8B, and described below.

Note there may be a single conductivity/temperature sensor, or a pair of conductivity/temperature sensors as shown. A pair of conductivity/temperature sensors may provide a check against poor accuracy or failure of one of the sensors.

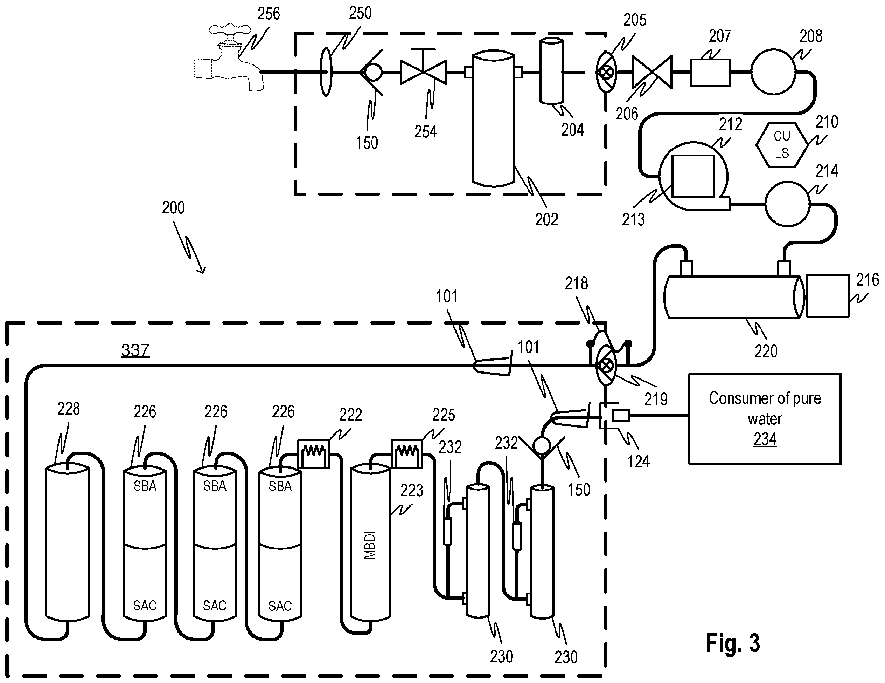

Fig. 3 shows a water treatment plant 200 that may constitute an embodiment the purified water source 133. The water treatment plant 200 has an initial pretreatment stage that includes a connector 250 to connect to an unfiltered water source 256, for example a water tap. The water flows through a check valve 150, through a pressure regulator 254, and then through a sediment filter 202. The check valve 150 prevents backflow of the water. The water then flows through an air vent 204 that removes air from the water. The water then flows through a connector 205 that connects to a water shutoff clamp 206, a snubber 207, and a water inlet pressure sensor 208. Water is pumped by water pump 212 which has an encoder 213 for precise tracking of the water pump 212 speed. The snubber 207 reduces pressure fluctuations. The water then flows through a water output pressure sensor 214, through an ultraviolet light lamp 220 and into a filter plant 337 that performs deionization, carbon filtration, and sterilizing filtration. A UV (ultraviolet) light sensor 216 may be provided to detect whether the ultraviolet light lamp 220 is operating, so that it can be replaced if it becomes inoperable. A first-use-fuse 218 together with a connector 219 is provided on the inlet of sterilizing filter plant 337, such that the fuse indicates whether the filter plant 337 has been used. This helps reduce the likelihood that a previously-used filter plant is reused unintentionally. A combined control unit and leak sensor are indicated at 210. In the sterilizing filter plant 337, the water flows through a carbon filter 228 and three separated bed deionization filters 226 which may be resin separated bed filters. The water then flows through a mixed bed deionization filter 223, which follows the separated bed filters 226. The mixed bed deionization filter 223 may be a resin mixed bed filter. Thereafter, the water flows through first and second ultrafilters 230, which follow the mixed bed deionization filter 223, and into the consumer of pure water 234. The embodiments of Figs. 1B and 1A are examples of a consumer of pure water 234.

Between a last separated bed deionization filter 226 and the mixed bed deionization filter 223 is a resistivity sensor 222 which indicates when the separated bed deionization filters 226 are nearing exhaustion, or at exhaustion. The mixed bed deionization filter 223 is still able to hold a predefined minimum magnitude of resistivity but the separated bed deionization filters 226 and the mixed bed deionization filter 223 may be replaced at the same time. In embodiments, along with the separated bed deionization filters 226 and the mixed bed deionization filter 223, the carbon filter 228 and ultrafilters 230 along with the interconnecting lines and other components may also be replaced as a single package. A current treatment can be completed in reliance on the mixed bed deionization filter 223 before the exhausted filters are replaced. A further resistivity sensor 225 detects unexpected problems with the separated bed deionization filter 223 upstream deionization filters which may require shutdown of the treatment and immediate replacement of the filters. Note that each of the ultrafilters 230 has an air vent 232. A check valve 150 is located downstream of the ultrafilters 230. The consumer of pure water 234 may be unit such as that of Figs. 1B or 1A which mixes a batch of medicament for use by a medicament user 157 such as a peritoneal dialysis cycler or any other type of medicament consuming device.

It should be evident from the above that the procedures of Fig. 2B in combination with those of Fig. 2A may be performed using the embodiments of Figs. 1B or 1A.

Note in any of the embodiments where the term clamp is used, it should be recognized that the functional element includes a tube or other flexible conduit and the clamp so that it functions as a valve. In any of the embodiments, another type of valve may be substituted for the clamp and conduit to provide the same function. Such a variation may be considered to alternative embodiments and clamp and conduit are not limiting of the subject matter conveyed herein.

Note that in any of the embodiments that identify the bag as the container, any bag may be replaced by any container including those of glass, polymer and other materials. In any embodiment where flow control is performed by a clamp, it should be understood that in any embodiment, including the claims, any clamp can be replaced by another type of valve such as a stopcock valve, a volcano valve, a ball valve, a gate valve or other type of flow controller. It should be understood that a clamp in the context of the disclosed subject matter is a clamp that closes around a tube to selectively control flow through the position of the clamp. Note that in any of the embodiments, the order of adding and mixing to the mixing container 102 can by reversed from what is described with respect to the embodiments. In any of the embodiments instead of dextrose concentrate being used, this can be substituted for glucose or another osmotic agent.

The process of providing purified water from the purified water source 133 is described next. As shown in Fig. 4A, water inlet clamp 138 is opened and the water pump 113 operates to convey purified water along water line 142. All other controllable valves (136, 139, 140, 306, 307) are closed. Water pump 113 together with the peristaltic pump 129 conveys water into the mixing container 102 through line 147 to line 125 as illustrated by the block arrows in Fig. 4A.

Line 125 can be provided with a check valve 154 (Fig. 1A) which prevents flow in one direction and has a cracking pressure which must be overcome for water to flow in the other direction. In the example of Fig. 1A, the check valve 154 permits water to flow through line 125 toward the mixing container 102 when the cracking pressure of the check valve 154 is overcome. Initially the purified water from the purified water source 133 is pumped by the water pump 113 with water inlet clamp 138 open and the batch release clamp 136 and the conductivity sensor clamp 140 closed such that water is pumped into the mixing container 102 through line 123 with the peristaltic pump 129 running so as to convey water into the mixing container 102, as shown in Fig. 4A.

Fig. 5A illustrates the configuration of the system when the osmotic agent concentrate 310 (e.g., dextrose, glucose, etc.) flows through osmotic supply line 312 and eventually into the mixing container 102. Even though Fig. 5A illustrates a fluid circuit configuration that corresponds to Fig. 1B, this description is equally applicable to the configuration shown in Fig. 1A. As shown in Fig. 5A, valve 306 is opened (all other valves remain closed) and peristaltic pump 129 can operate in in the direction shown (pictured to the right on the drawing sheet), such that the osmotic concentrate 310 flows through inlet line 123 into mixing container 102. The peristaltic pump 129 can be controlled to precisely meter a desired quantity of the osmotic concentrate into mixing container 102. In embodiments, only a fraction of the total quantity of the osmotic agent concentrate 310 present in its container is provided into mixing container 102, such that multiple batches of the medicament can be prepared in the mixing container 102; and each of the batches can be customized based on a desired concentration to create custom mini batches.

The osmotic agent concentrate 310 passes through the sterilizing filter 630 on its way to the mixing container 102, thereby minimizing the risk of contamination.

In an alternate embodiment, the osmotic concentrate 310 can be positioned sufficiently high or above mixing container 102 that a gravity powered fill can be accomplished (if the cracking pressure of the check valve 154 can be overcome). In this scenario, valve 306 is opened and valve 139 is opened (not illustrated in Fig. 5A) which permits gravity to convey the osmotic agent concentrate through inlet line 125 into mixing container 102, without the use of peristaltic pump 129. In embodiments, the entirety of the osmotic agent concentrate 310 is allowed to flow into the mixing container 102 so that the quantity of the osmotic agent concentrate 310 that is present in the mixing container 102 is known based on the original amount of the osmotic agent concentrate that was present in its initial container.

Fig. 5B illustrates the configuration of the system when the electrolyte concentrate 316 flows through electrolyte supply line 318 and eventually into the mixing container 102. As shown in Fig. 5B, valve 307 is opened (with all other valves closed) and peristaltic pump 129 can operate in in the direction shown (pictured to the right on the drawing sheet), such that the electrolyte concentrate 316 flows through inlet line 123 into mixing container 102. The electrolyte concentrate 310 passes through the sterilizing filter 630 on its way to the mixing container 102, thereby minimizing the risk of contamination.

The peristaltic pump 129 can be controlled to precisely meter a desired quantity of the electrolyte concentrate into mixing container 102. In embodiments, only a fraction of the total quantity of the electrolyte concentrate 316 present in its container is provided into mixing container 102, such that multiple batches of the medicament can be prepared in the mixing container 102; and each of the batches can be customized based on a desired concentration to create custom mini batches.

In an alternate embodiment, the electrolyte concentrate 316 can be positioned sufficiently high or above mixing container 102 that a gravity powered fill can be accomplished. In this scenario, valve 307 is opened and valve 139 is opened which permits gravity to convey the electrolyte concentrate through inlet line 125 into mixing container 102, without the use of peristaltic pump 129 (of the cracking pressure of the check valve 154 can be overcome). In embodiments, the entirety of the electrolyte concentrate 316 is allowed to flow into the mixing container 102 so that the quantity of the electrolyte concentrate 316 that is present in the mixing container 102 is known based on the original amount of the electrolyte concentrate that was present in its initial container.

Referring to Fig. 6A, to mix the contents of the mixing container 102 the peristaltic pump 129 pumps fluid in a circular path through lines 123, 125, and 149 in a direction designated with an arrow in the figure (to the left in the figure) with all the clamps closed except for clamp 139. The peristaltic pump 129 generates sufficient pressure to overcome the cracking pressure of check valve 154. Then the contents of the mixing container 102 are mixed by the flow circulating through the mixing container 102. While the contents of the mixing container 102 circulate through the fluid path that is illustrated, they pass through or past conductivity/temperature sensors 159c and 159 s, and the conductivity and/or the temperature of the fluid can be measured and the measurement provided to controller 141, as described above. The mixing process may continue for a predetermined period of time, or it may continue until the conductivity a value that is measured by the conductivity sensors reaches a stable value that does not change any more.

Referring to Fig. 7, a configuration of the fluid circuit that is used for draining the mixing container is shown. Drain valve 140 is opened with all other valves remaining closed, and the peristaltic pump 129 operates in the direction as shown which causes the contents of the mixing container to pass through inlet line 123 to transfer line 149, to drain line 147 and ultimately to drain connection 152. The mixing container 102 may be drained at the conclusion of a treatment cycle, or when it is determined that the contents of the mixing container is passed its expiration, and/or when the filter integrity test indicates a failure of filter 630. In embodiments, the contents of the mixing container 102 may also be drained when it is determined that the conductivity measurement is grossly incorrect.

Referring now to Fig. 8A and 8B, once of the medicament is prepared and mixed in the mixing container at 102, and the medicament is deemed to be ready for use based on conductivity checks described above and filter integrity test, the medicament is provided to the medicament user 157. Figs. 8A and 8B illustrate various arrangements of the fluid circuit for providing the medicament. At this time, the water inlet clamp 138 and the drain line clamp 140 are closed. The medicament user 157 may be any type of treatment device or container that receives the mixed medicament from the mixing container 102.

The batch release clamp 136 and valve 139 are open and the water inlet clamp 138 and the conductivity sensor clamp 140 are closed. A pump 115 in a medicament user 157 may then draw fluid from the circular path as the peristaltic pump 129 rotates to maintain fluid at the cracking pressure of the check valve 154 in Fig. 8A. In embodiments, the cracking pressure may be 3.5 PSI (pounds per square inch). It will be understood that this makes the medicament preparation system appear like a bag of dialysate with a head pressure of 3.5 PSI.

The medicament pump 115 in the medicament user 157 may see a positive pressure at the cracking pressure type check valve 154 cracking pressure which may facilitate the pump 115 of the medicament user 157 by mimicking the pressure of an elevated medicament container with a head pressure approximately at the cracking pressure of the check valve 154. In embodiments, as shown in Fig. 8B, clamp 139 is closed while peristaltic pump 129 operates in the direction shown in the drawing. Clamp 136 is opened, and the medicament is conveyed through medicament output lines 137 and 153 to medicament user 157. A pressure sensor 301 is provided to measure the pressure in this fluid channel and to provide a signal, which may be used in feedback control, to modulate the speed of the peristaltic pump 129 and thereby provide a predetermined pressure in the formed fluid channel.

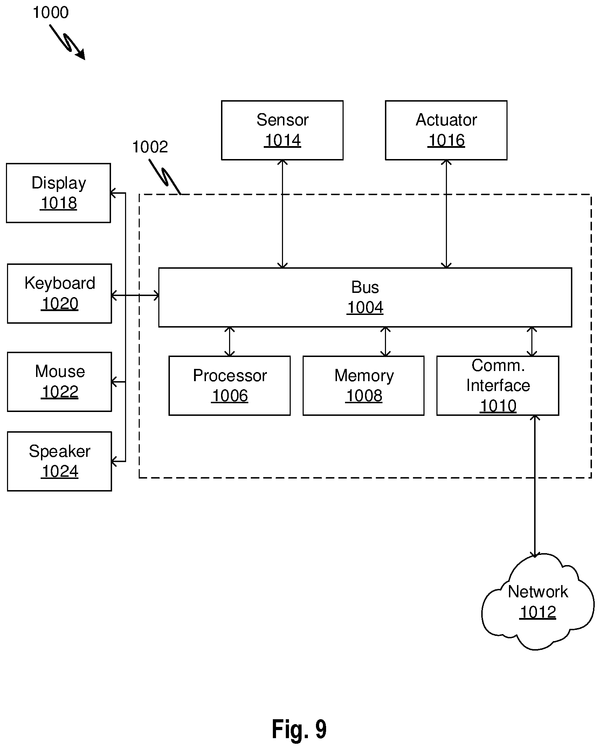

Fig. 9 shows a block diagram of an example computer system according to embodiments of the disclosed subject matter. In various embodiments, all, or parts of system 1000 may be included in a medical treatment device/system such as a renal replacement therapy system. In these embodiments, all, or parts of system 1000 may provide the functionality of a controller of the medical treatment device/systems. In some embodiments, all, or parts of system 1000 may be implemented as a distributed system, for example, as a cloud-based system.

System 1000 includes a computer 1002 such as a personal computer or workstation or other such computing system that includes a processor 1006. However, alternative embodiments may implement more than one processor and/or one or more microprocessors, microcontroller devices, or control logic including integrated circuits such as ASIC.

Computer 1002 further includes a bus 1004 that provides communication functionality among various modules of computer 1002. For example, bus 1004 may allow for communicating information/data between processor 1006 and a memory 1008 of computer 1002 so that processor 1006 may retrieve stored data from memory 1008 and/or execute instructions stored on memory 1008. In one embodiment, such instructions may be compiled from source code/objects provided in accordance with a programming language such as Java, C++, C#, .net, Visual Basic^{™} language, LabVIEW, or another structured or object-oriented programming language. In one embodiment, the instructions include software modules that, when executed by processor 1006, provide renal replacement therapy functionality according to any of the embodiments disclosed herein.

Memory 1008 may include any volatile or non-volatile computer-readable memory that can be read by computer 1002. For example, memory 1008 may include a non-transitory computer-readable medium such as ROM, PROM, EEPROM, RAM, flash memory, disk drive, etc. Memory 1008 may be a removable or non-removable medium.

Bus 1004 may further allow for communication between computer 1002 and a display 1018, a keyboard 1020, a mouse 1022, and a speaker 1024, each providing respective functionality in accordance with various embodiments disclosed herein, for example, for configuring a treatment for a patient and monitoring a patient during a treatment.

Computer 1002 may also implement a communication interface 1010 to communicate with a network 1012 to provide any functionality disclosed herein, for example, for alerting a healthcare professional and/or receiving instructions from a healthcare professional, reporting patient/device conditions in a distributed system for training a machine learning algorithm, logging data to a remote repository, etc. Communication interface 1010 may be any such interface known in the art to provide wireless and/or wired communication, such as a network card or a modem.

Bus 1004 may further allow for communication with one or more sensors 1014 and one or more actuators 1016, each providing respective functionality in accordance with various embodiments disclosed herein, for example, for measuring signals.

It will be appreciated that the modules, processes, systems, and sections described above can be implemented in hardware, hardware programmed by software, software instruction stored on a non-transitory computer readable medium or a combination of the above. For example, a method for providing a medicament to a medicament user can be implemented, for example, using a processor configured to execute a sequence of programmed instructions stored on a non-transitory computer readable medium. For example, the processor can include, but not be limited to, a personal computer or workstation or other such computing system that includes a processor, microprocessor, microcontroller device, or is comprised of control logic including integrated circuits such as, for example, an Application Specific Integrated Circuit (ASIC). The instructions can be compiled from source code instructions provided in accordance with a programming language such as Java, C++, C#.net or the like. The instructions can also comprise code and data objects provided in accordance with, for example, the Visual Basic^{™} language, LabVIEW, or another structured or object-oriented programming language. The sequence of programmed instructions and data associated therewith can be stored in a non-transitory computer-readable medium such as a computer memory or storage device which may be any suitable memory apparatus, such as, but not limited to read-only memory (ROM), programmable read-only memory (PROM), electrically erasable programmable read-only memory (EEPROM), random-access memory (RAM), flash memory, disk drive and the like.

Furthermore, the modules, processes, systems, and sections can be implemented as a single processor or as a distributed processor. Further, it should be appreciated that the steps mentioned above may be performed on a single or distributed processor (single and/or multicore). Also, the processes, modules, and sub-modules described in the various figures of and for embodiments above may be distributed across multiple computers or systems or may be co-located in a single processor or system. Exemplary structural embodiment alternatives suitable for implementing the modules, sections, systems, means, or processes described herein are provided below.

The modules, processors or systems described above can be implemented as a programmed general purpose computer, an electronic device programmed with microcode, a hard-wired analog logic circuit, software stored on a computer-readable medium or signal, an optical computing device, a networked system of electronic and/or optical devices, a special purpose computing device, an integrated circuit device, a semiconductor chip, and a software module or object stored on a computer-readable medium or signal, for example.

Embodiments of the method and system (or their sub-components or modules), may be implemented on a general-purpose computer, a special-purpose computer, a programmed microprocessor or microcontroller and peripheral integrated circuit element, an ASIC or other integrated circuit, a digital signal processor, a hardwired electronic or logic circuit such as a discrete element circuit, a programmed logic circuit such as a programmable logic device (PLD), programmable logic array (PLA), field-programmable gate array (FPGA), programmable array logic (PAL) device, or the like. In general, any process capable of implementing the functions or steps described herein can be used to implement embodiments of the method, system, or a computer program product (software program stored on a non-transitory computer readable medium).

Furthermore, embodiments of the disclosed method, system, and computer program product may be readily implemented, fully or partially, in software using, for example, object or object-oriented software development environments that provide portable source code that can be used on a variety of computer platforms. Alternatively, embodiments of the disclosed method, system, and computer program product can be implemented partially or fully in hardware using, for example, standard logic circuits or a very-large-scale integration (VLSI) design. Other hardware or software can be used to implement embodiments depending on the speed and/or efficiency requirements of the systems, the particular function, and/or particular software or hardware system, microprocessor, or microcomputer being utilized. Embodiments of the method, system, and computer program product can be implemented in hardware and/or software using any known or later developed systems or structures, devices and/or software by those of ordinary skill in the applicable art from the function description provided herein and with a general basic knowledge of control systems of medical devices and/or computer programming arts.

Moreover, embodiments of the disclosed method, system, and computer program product can be implemented in software executed on a programmed general-purpose computer, a special purpose computer, a microprocessor, or the like.

It is, thus, apparent that there is provided, in accordance with the present disclosure, Medicament Preparation Devices, Methods, and Systems. Furthermore, certain features may sometimes be used to advantage without a corresponding use of other features.

## Claims

1. A method of generating a custom mini batch of dialysate with a proportioning system, the method comprising:
attaching a disposable component (161) to the proportioning system;
generating purified water with a water purification system (133, 200);
adding a first quantity of the purified water to a mixing container (102) that is pre-attached to the disposable component (161);
conveying a second quantity of a first concentrated medicament through a sterilizing filter (630) and to the mixing container (102);
first mixing contents of the mixing container (102);
determining a concentration of the contents of the mixing container (102) by flowing the contents past at least one fluid quality sensor and back into the mixing container (102);
conveying a third quantity of a second concentrated medicament through the sterilizing filter (630) and to the mixing container (102);
second mixing the contents of the mixing container (102);
confirming a final concentration of the contents of the mixing container (102) by flowing the contents past at least one fluid quality sensor and back into the mixing container (102);
testing integrity of the sterilizing filter (630) ; and
if the testing indicates that the sterilizing filter (630) is acceptable, providing the contents of the mixing container (102) to a medicament user (157), and otherwise indicating an error condition.

2. The method according to claim 1, wherein the determining of the concentration of the contents of the mixing container (102) includes continuously recirculating at least a portion of the contents of the mixing container (102) past the at least one fluid quality sensor until a value output by the fluid quality sensor reaches a steady state.

3. The method according to claim 1, wherein the determining of the concentration of the contents of the mixing container (102) includes flowing at least a portion of the contents of the mixing container (102) past two conductivity sensors (159c, 159s) until a value output from the two conductivity sensors (159c, 159s) reaches a steady state value.

4. The method according to claim 1, further comprising:
connecting a first source (310) of the first concentrated medicament to the disposable component (161) with a connector (312); and
connecting a second source (316) of the second concentrated medicament to the disposable component (161) with a second connector (318).

5. The method according to claim 1, wherein the determining of the concentration of the contents of the mixing container (102) includes measuring a conductivity of the contents.

6. The method according to claim 1, further comprising:
conveying a variable quantity of the purified water to the mixing container (102) after the first mixing, wherein the variable quantity is determined based on the determined concentration of the contents.

7. The method according to claim 6, further comprising:
further determining a concentration of the contents at a time after the conveying of the variable quantity of the purified water to the mixing container (102) and before the conveying of the third quantity of the second concentrated medicament to the mixing container (102).

8. The method according to claim 6, wherein the variable quantity of the purified water is less than a difference between the first quantity of the purified water and an estimated total quantity of the purified water required in the custom mini batch of dialysate.

9. The method according to claim 1, further comprising:
conveying a second variable quantity of the purified water to the mixing container (102) after the second mixing, wherein the second variable quantity is determined based on the determined concentration of the contents of the mixing container (102) after the second mixing.

10. The method according to claim 1, wherein the providing of the contents of the mixing container (102) to the medicament user (157) takes place less than an hour after an initiation of production of the custom mini batch of dialysate.

11. The method according to claim 1, wherein the testing of the integrity of the sterilizing filter (630) is performed at any one or more of a first time that is after the conveying of the second quantity of the first concentrated medicament to the mixing container (102) and before the first mixing of the contents of the mixing container (102), a second time that is after the first mixing of the contents of the mixing container (102) and before the conveying of the third quantity of the second concentrated medicament to the mixing container (102), and a third time that is after the conveying of the third quantity of the second concentrated medicament to the mixing container (102) and before the second mixing of the contents of the mixing container (102).

12. The method according to claim 1, wherein the testing of the integrity of the sterilizing filter (630) is performed after the second mixing of the contents of the mixing container (102).

13. The method according to claim 1, wherein the testing of the integrity of the sterilizing filter (630) includes performing a pressurized air test on a membrane of the sterilizing filter (630).

14. The method according to claim 1, wherein the conveying of the third quantity of the second concentrated medicament through the sterilizing filter (630) and to the mixing container (102) comprises conveying the third quantity of the second concentrated medicament through the sterilizing filter (630) and to the mixing container (102) in response to the determining of the concentration of the contents indicating that there is no gross error in a measurement of the concentration of the contents.

15. The method according to claim 1, wherein the first concentrated medicament is an osmotic agent concentrate and the second concentrated medicament is an electrolyte concentrate.

## Patentansprüche

1. Verfahren zum Erzeugen einer benutzerdefinierten Kleincharge Dialysat mit einem Dosiersystem, wobei das Verfahren Folgendes umfasst:
Anbringen eines Einweg-Bauteils (161) an dem Dosiersystem;
Erzeugen gereinigten Wassers mit einem Wasserreinigungssystem (133, 200);
Hinzufügen einer ersten Menge des gereinigten Wassers zu einem Mischbehälter (102), welcher zuvor an dem Einweg-Bauteil (161) angebracht wurde;
Befördern einer zweiten Menge eines ersten konzentrierten Medikaments durch einen Sterilisierfilter (630) und zu dem Mischbehälter (102);
erstes Mischen von Inhalt des Mischbehälters (102);
Bestimmen einer Konzentration des Inhalts des Mischbehälters (102) durch Veranlassen, dass der Inhalt an mindestens einem Fluidqualitätssensor vorbei und zurück in den Mischbehälter (102) fließt;
Befördern einer dritten Menge eines zweiten konzentrierten Medikaments durch den Sterilisierfilter (630) und zu dem Mischbehälter (102);
zweites Mischen des Inhalts des Mischbehälters (102);
Bestätigen einer finalen Konzentration des Inhalts des Mischbehälters (102) durch Veranlassen, dass der Inhalt an mindestens einem Fluidqualitätssensor vorbei und zurück in den Mischbehälter (102) fließt;
Testen einer Unversehrtheit des Sterilisierfilters (630); und,
wenn das Testen anzeigt, dass der Sterilisierfilter (630) akzeptabel ist, Abgeben des Inhalts des Mischbehälters (102) an einen Medikamentenanwender (157) und anderenfalls Anzeigen eines Fehlerzustands.

2. Verfahren nach Anspruch 1, wobei das Bestimmen der Konzentration des Inhalts des Mischbehälters (102) umfasst, mindestens einen Teil des Inhalts des Mischbehälters (102) kontinuierlich an dem mindestens einen Fluidqualitätssensor vorbei im Kreislauf umzupumpen, bis ein von dem Fluidqualitätssensor ausgegebener Wert einen stabilen Zustand erreicht.

3. Verfahren nach Anspruch 1, wobei das Bestimmen der Konzentration des Inhalts des Mischbehälters (102) umfasst, zu veranlassen, dass mindestens ein Teil des Inhalts des Mischbehälters (102) an zwei Leitfähigkeitssensoren (159c, 159s) vorbeifließt, bis ein von den zwei Leitfähigkeitssensoren (159c, 159s) ausgegebener Wert einen stabilen Zustandswert erreicht.

4. Verfahren nach Anspruch 1, ferner umfassend:
Verbinden einer ersten Quelle (310) des ersten konzentrierten Medikaments mit dem Einweg-Bauteil (161) mittels eines Verbinders (312); und
Verbinden einer zweiten Quelle (316) des zweiten konzentrierten Medikaments mit dem Einweg-Bauteil (161) mittels eines zweiten Verbinders (318).

5. Verfahren nach Anspruch 1, wobei das Bestimmen der Konzentration des Inhalts des Mischbehälters (102) Messen einer Leitfähigkeit des Inhalts umfasst.

6. Verfahren nach Anspruch 1, ferner umfassend:
Befördern einer variablen Menge des gereinigten Wassers zu dem Mischbehälter (102) nach dem ersten Mischen, wobei die variable Menge basierend auf der bestimmten Konzentration des Inhalts bestimmt wird.

7. Verfahren nach Anspruch 6, ferner umfassend:
ferner Bestimmen einer Konzentration des Inhalts zu einem Zeitpunkt nach dem Befördern der variablen Menge des gereinigten Wassers zu dem Mischbehälter (102) und vor dem Befördern der dritten Menge des zweiten konzentrierten Medikaments zu dem Mischbehälter (102).

8. Verfahren nach Anspruch 6, wobei die variable Menge des gereinigten Wassers geringer ist als eine Differenz zwischen der ersten Menge des gereinigten Wassers und einer geschätzten bei der benutzerdefinierten Kleincharge Dialysat erforderlichen Gesamtmenge des gereinigten Wassers.

9. Verfahren nach Anspruch 1, ferner umfassend:
Befördern einer zweiten variablen Menge des gereinigten Wassers zu dem Mischbehälter (102) nach dem zweiten Mischen, wobei die zweite variable Menge basierend auf der bestimmten Konzentration des Inhalts des Mischbehälters (102) nach dem zweiten Mischen bestimmt wird.

10. Verfahren nach Anspruch 1, wobei das Abgeben des Inhalts des Mischbehälters (102) an den Medikamentenanwender (157) weniger als eine Stunde nach einem Herstellungsbeginn der benutzerdefinierten Kleincharge Dialysat stattfindet.

11. Verfahren nach Anspruch 1, wobei das Testen der Unversehrtheit des Sterilisierfilters (630) zu einem ersten Zeitpunkt, das heißt nach dem Befördern der zweiten Menge des ersten konzentrierten Medikaments zu dem Mischbehälter (102) und vor dem ersten Mischen des Inhalts des Mischbehälters (102), und/oder zu einem zweiten Zeitpunkt, das heißt nach dem ersten Mischen des Inhalts des Mischbehälters (102) und vor dem Befördern der dritten Menge des zweiten konzentrierten Medikaments zu dem Mischbehälter (102), und/oder zu einem dritten Zeitpunkt, das heißt nach dem Befördern der dritten Menge des zweiten konzentrierten Medikaments zu dem Mischbehälter (102) und vor dem zweiten Mischen des Inhalts des Mischbehälters (102), durchgeführt wird.

12. Verfahren nach Anspruch 1, wobei das Testen der Unversehrtheit des Sterilisierfilters (630) nach dem zweiten Mischen des Inhalts des Mischbehälters (102) durchgeführt wird.

13. Verfahren nach Anspruch 1, wobei das Testen der Unversehrtheit des Sterilisierfilters (630) Durchführen eines Druckufttests an einer Membran des Sterilisierfilters (630) umfasst.

14. Verfahren nach Anspruch 1, wobei das Befördern der dritten Menge des zweiten konzentrierten Medikaments durch den Sterilisierfilter (630) und zu dem Mischbehälter (102) Befördern der dritten Menge des zweiten konzentrierten Medikaments durch den Sterilisierfilter (630) und zu dem Mischbehälter (102) als Reaktion darauf, dass das Bestimmen der Konzentration des Inhalts anzeigt, dass kein grober Fehler bei einer Messung der Konzentration des Inhalts vorliegt, umfasst.

15. Verfahren nach Anspruch 1, wobei das erste konzentrierte Medikament ein Osmosemittelkonzentrat ist und das zweite konzentrierte Medikament ein Elektrolytkonzentrat ist.

## Revendications

1. Procédé de génération d'un mini-lot personnalisé de dialysat avec un système de dosage, le procédé comprenant :
la fixation d'un composant jetable (161) au système de dosage ;
la génération d'eau purifiée avec un système de purification d'eau (133, 200) ;
l'ajout d'une première quantité de l'eau purifiée à un récipient de mélange (102) qui est pré-fixé au composant jetable (161) ;
l'acheminement d'une deuxième quantité d'un premier médicament concentré à travers un filtre de stérilisation (630) et vers le récipient de mélange (102) ;
le premier mélange du contenu du récipient de mélange (102) ;
la détermination d'une concentration du contenu du récipient de mélange (102) en faisant s'écouler le contenu devant au moins un capteur de qualité de fluide et de retour dans le récipient de mélange (102) ;
l'acheminement d'une troisième quantité d'un second médicament concentré à travers le filtre de stérilisation (630) et vers le récipient de mélange (102) ;
le second mélange du contenu du récipient de mélange (102) ;
la confirmation d'une concentration finale du contenu du récipient de mélange (102) en faisant s'écouler le contenu devant au moins un capteur de qualité de fluide et de retour dans le récipient de mélange (102) ;
le test d'intégrité du filtre de stérilisation (630) ; et
si le test indique que le filtre de stérilisation (630) est acceptable, la fourniture du contenu du récipient de mélange (102) à un utilisateur de médicament (157), et sinon l'indication d'une condition d'erreur.

2. Procédé selon la revendication 1, la détermination de la concentration du contenu du récipient de mélange (102) comprenant la recirculation continue d'au moins une partie du contenu du récipient de mélange (102) devant l'au moins un capteur de qualité de fluide jusqu'à ce qu'une valeur émise par le capteur de qualité de fluide atteigne un état stable.

3. Procédé selon la revendication 1, la détermination de la concentration du contenu du récipient de mélange (102) comprenant l'écoulement d'au moins une partie du contenu du récipient de mélange (102) devant deux capteurs de conductivité (159c, 159s) jusqu'à ce qu'une valeur émise par les deux capteurs de conductivité (159c, 159s) atteigne une valeur d'état stable.

4. Procédé selon la revendication 1, comprenant en outre :
la connexion d'une première source (310) du premier médicament concentré au composant jetable (161) avec un connecteur (312) ; et
la connexion d'une seconde source (316) du second médicament concentré au composant jetable (161) avec un second connecteur (318).

5. Procédé selon la revendication 1, la détermination de la concentration du contenu du récipient de mélange (102) comprenant la mesure d'une conductivité du contenu.

6. Procédé selon la revendication 1, comprenant en outre :
l'acheminement d'une quantité variable de l'eau purifiée vers le récipient de mélange (102) après le premier mélange, la quantité variable étant déterminée sur la base de la concentration déterminée du contenu.

7. Procédé selon la revendication 6, comprenant en outre :
la détermination supplémentaire d'une concentration du contenu à un moment après l'acheminement de la quantité variable de l'eau purifiée vers le récipient de mélange (102) et avant l'acheminement de la troisième quantité du second médicament concentré vers le récipient de mélange (102).

8. Procédé selon la revendication 6, la quantité variable de l'eau purifiée étant inférieure à une différence entre la première quantité de l'eau purifiée et une quantité totale estimée de l'eau purifiée requise dans le mini-lot personnalisé de dialysat.

9. Procédé selon la revendication 1, comprenant en outre :
l'acheminement d'une deuxième quantité variable de l'eau purifiée vers le récipient de mélange (102) après le second mélange, la deuxième quantité variable étant déterminée sur la base de la concentration déterminée du contenu du récipient de mélange (102) après le second mélange.

10. Procédé selon la revendication 1, la fourniture du contenu du récipient de mélange (102) à l'utilisateur de médicament (157) ayant lieu moins d'une heure après une initiation de production du mini-lot personnalisé de dialysat.

11. Procédé selon la revendication 1, le test de l'intégrité du filtre de stérilisation (630) étant effectué à n'importe lequel d'un ou plusieurs d'un premier moment qui est après l'acheminement de la deuxième quantité du premier médicament concentré vers le récipient de mélange (102) et avant le premier mélange du contenu du récipient de mélange (102), d'un deuxième moment qui est après le premier mélange du contenu du récipient de mélange (102) et avant l'acheminement de la troisième quantité du second médicament concentré vers le récipient de mélange (102), et d'un troisième moment qui est après l'acheminement de la troisième quantité du second médicament concentré vers le récipient de mélange (102) et avant le second mélange du contenu du récipient de mélange (102).

12. Procédé selon la revendication 1, le test de l'intégrité du filtre de stérilisation (630) étant effectué après le second mélange du contenu du récipient de mélange (102).

13. Procédé selon la revendication 1, le test de l'intégrité du filtre de stérilisation (630) comprenant la réalisation d'un test d'air sous pression sur une membrane du filtre de stérilisation (630).

14. Procédé selon la revendication 1, l'acheminement de la troisième quantité du second médicament concentré à travers le filtre de stérilisation (630) et vers le récipient de mélange (102) comprenant l'acheminement de la troisième quantité du second médicament concentré à travers le filtre de stérilisation (630) et vers le récipient de mélange (102) en réponse à la détermination de la concentration du contenu indiquant qu'il n'y a aucune erreur grossière dans une mesure de la concentration du contenu.

15. Procédé selon la revendication 1, le premier médicament concentré étant un concentré d'agent osmotique et le second médicament concentré étant un concentré d'électrolyte.
